(19) **Europäisches Patentamt / European Patent Office / Office européen des brevets**

(11) **EP 2 550 531 B1**

(12) **EUROPEAN PATENT SPECIFICATION**

(45) Date of publication and mention of the grant of the patent:
**31.05.2017 Bulletin 2017/22**

(21) Application number: **11709648.7**

(22) Date of filing: **21.03.2011**

(51) Int Cl.:
*G01N 33/49* (2006.01)     *G01N 33/72* (2006.01)

(86) International application number:
**PCT/EP2011/001387**

(87) International publication number:
**WO 2011/116919 (29.09.2011 Gazette 2011/39)**

(54) **METHODS AND APPARATUSES FOR PREDICTING THE EFFECTS OF ERYTHROPOESIS STIMULATING AGENTS (ESA), AND FOR DETERMINING A DOSE TO BE ADMINISTERED**

VERFAHREN UND VORRICHTUNGEN ZUR VORHERSAGE DER EFFEKTE VON ERYTHROPOESESTIMULIERENDE SUBSTANZEN (ESA) UND ZUR BESTIMMUNG DER ZU VERABREICHENDEN DOSIS

PROCÉDÉ ET APPAREIL POUR PRÉDIRE LES EFFETS D'AGENTS DE STIMULATION DE L'ÉRYTHROPOÏÈSE ET POUR DÉTERMINER LA DOSE À ADMINISTRER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2010 US 370130 P**
**03.08.2010 EP 10008085**
**23.03.2010 EP 10003050**

(43) Date of publication of application:
**30.01.2013 Bulletin 2013/05**

(73) Proprietor: **Fresenius Medical Care Deutschland GmbH**
**61352 Bad Homburg (DE)**

(72) Inventors:
- **CHAMNEY, Paul**
  **Tring**
  **HP23 4DZ (GB)**
- **MOISSL, Ulrich**
  **61184 Karben (DE)**
- **WABEL, Peter**
  **64287 Darmstadt (DE)**
- **WIESKOTTEN, Sebastian**
  **64560 Erfelden (DE)**
- **NIER, Volker**
  **61203 Reicheisheim (DE)**

(74) Representative: **Bobbert & Partner Patentanwälte PartmbB**
**Postfach 1252**
**85422 Erding (DE)**

(56) References cited:
**EP-A2- 0 419 223          EP-A2- 0 419 223**
**WO-A1-92/19153          WO-A1-03/045423**
**WO-A1-03/045423          WO-A1-2006/002685**
**WO-A1-2006/002685          WO-A2-02/10725**
**WO-A2-02/10725          WO-A2-2007/033318**
**WO-A2-2007/033318          WO-A2-2010/053626**
**WO-A2-2010/053626          US-A1- 2003 198 691**
**US-A1- 2003 198 691**

- **PORT R E ET AL: "Predicting the time course of haemoglobin in children treated with erythropoietin for renal anaemia", BRITISH JOURNAL OF CLINICAL PHARMACOLOGY, vol. 46, no. 5, November 1998 (1998-11), pages 461-466, XP002632457, ISSN: 0306-5251**
- **PORT R E ET AL: "Predicting the time course of haemoglobin in children treated with erythropoietin for renal anaemia", BRITISH JOURNAL OF CLINICAL PHARMACOLOGY, BLACKWELL SCIENTIFIC PUBL, GB, vol. 46, 1 January 1998 (1998-01-01), pages 461-466, XP002632457, ISSN: 0306-5251**
- **"Hemodialysis machines and monitors" In: DRUKKER, PARSON, MAHER: "Replacement of Renal Function by Dialysis", KLUWER ACADEMIC PUBLISHER pages 397-401,**

**Description**

The present invention relates to a computer implemented method for predicting or assessing the concentration or the mass of haemoglobin or an approximation thereof, respectively, in a body fluid sample and/or an extracorporeal sample thereof of a patient at a later, second point of time, the patient having theoretically or in reality been administered a certain dose of an erythropoesis stimulating agent at an earlier, first point of time according to the preamble of claim 1, and/or according to claims 2 and 3. It relates further to corresponding devices according to claims 4-6. Finally the present invention relates to digital storage means, a computer program product, and a computer program, and to a method for treating anemia according to claim 7. In certain situations the mass or the concentration of a haemoglobin (Hb, also known as Hgb, being the iron-containing oxygen-transport metalloprotein in the red blood cells) that is present in a patient's body has to be checked or monitored, e. g., by the physician in charge, for being in a position to determine as appropriately as possible the dose or dosage (both terms being used as synonyms of the term 'dosage' within the present specification) of an erythropoesis stimulating agent (ESA) to be administered.

In practise, the concentration of haemoglobin is measured by means of blood samples to assess the anemia state of the patient. Values below given thresholds are usually considered as a sign for the manifestation of "anemia" being defined as a decrease in normal number of red blood cells (RBCs) or less than the normal quantity of haemoglobin in the blood. Based on these findings, the dose or dosage of an erythropoesis stimulating agent (ESA) is determined, calculated or fixed. Since for certain reasons such as medical and also economical reasons the dose of ESA should be adapted to need as well as possible, it is of high interest to know in advance how ESA, once administered, will effect the future haemoglobin concentration.

However, maintaining the haemoglobin (Hb) stable within defined guidelines by exogenous doses of erythropoietin (EPO) and iron is difficult to achieve in the clinical setting.

When there is a change in the dose of erythropoietin (EPO) administered, the number of red blood cells begins to change almost immediately. If the correct amount of iron can be delivered to the production of red cells, the haemoglobin mass will change almost immediately. However, the erythrocyte mass does not achieve steady state until a period of time has elapsed which depends on the erythrocyte lifetime (typically 120 days in health), hereinafter known as the "response time". The erythrocyte lifetime may be shorter in various disease conditions, especially in the presence of inflammatory conditions.

Hb is usually monitored in clinical practice by blood sampling on a monthly basis. If, one month after administering the new EPO dose, the value of Hb is found to be outside the target or target range, the EPO dose is often changed again. By making changes to the EPO dose before the end of the time needed for a response, not only does the value of Hb tend to oscillate, but the Hb value can oscillate to values outside the target range.

From a practical perspective a great deal of clinical resource is bound continually monitoring Hb levels in blood and by adjusting EPO doses. Much of this resource could be saved by automatic monitoring of Hb with suitable technology and methods to find the optimal dose of EPO to maintain stable Hb concentration values over a period of months.

Therefore, by means of the present disclosure a method for predicting or assessing the concentration or the mass of haemoglobin and a method for determining the dose of an erythropoesis stimulating agent (ESA) which includes EPO and iron dosing to be administered for achieving a target Hb concentration or mass is suggested. Also, devices for carrying out the methods according to the invention are provided, as well as digital storage means, a computer program product, and a computer program. Finally, an erythropoesis stimulating medicament for use according to a certain administration scheme is proposed.

The method for predicting or assessing according to the invention is defined by the feature combination of claim 1. Accordingly, in one aspect of the invention, a method for predicting the concentration or the mass of haemoglobin or an approximation thereof, respectively, in a body fluid sample of a patient and/or an extracorporeal sample thereof, such as a blood sample, at a later, second point of time is suggested, the patient having theoretically or in reality been administered a certain, i. e., known, dose of an erythropoesis stimulating agent at an earlier, first point of time.

The method comprises the step of partially or completely correcting a measured or calculated haemoglobin mass or concentration value or an approximation thereof, which can be measured at the first or any other point of time such as a third point of time, respectively, for an overhydration or hyperhydration, respectively, of the patient or for parts of the overhydration, or its distortion effected by the overhydration, in order to get a corrected Hb mass or concentration value.

The method further comprises the step of predicting the haemoglobin mass or concentration at the second point of time starting from or based on the corrected mass or concentration value.

The apparatus or device according to the invention for prediction is defined by the feature combination of claim 4. Accordingly, in another aspect of the invention, a device for predicting the concentration or the mass of haemoglobin or an approximation thereof, respectively, in a body fluid sample a patient and/or an extracorporeal sample thereof at a later, second point of time, the patient having theoretically or in reality been administered a certain dose of an erythropoesis stimulating agent at an earlier, first point of time, is proposed. The device comprises a correction means configured for computationally correcting a measured haemoglobin mass or concentration value or an approximation thereof, respectively, for an overhydration of the patient in order to get a corrected haemoglobin (Hb) mass or concentration. It also comprises a prediction means configured for predicting the haemoglobin mass or concentration at the second point of time starting from the corrected mass or concentration value.

Accordingly, in another aspect of the disclosure, a method for determining the dose of an erythropoesis stimulating agent to be administered to a patient at an earlier, first point of time, for being in a position to find a desired concentration or the mass of haemoglobin an approximation thereof (also referred to as target concentration or mass), respectively, in a body fluid of the patient and/or an extracorporeal sample thereof at a later, second point of time, is suggested. The method encompasses the step of correcting a measured haemoglobin mass or concentration value or an approximation thereof, respectively, for or by an overhydration of the patient in order to get a corrected mass or concentration. It further comprises the step of determining the dose of the agent to be administered starting from the corrected haemoglobin mass or concentration value.

Accordingly, in another aspect of the disclosure, the device for determining the dose of an erythropoesis stimulating agent to be administered to a patient at an earlier, first point of time, for being in a position to find a desired concentration or the mass of haemoglobin an approximation thereof, respectively, in a body fluid of a patient and/or an extracorporeal sample thereof at a later, second point of time, comprises a correction means configured for correcting a measured haemoglobin mass or concentration value or an approximation thereof, respectively, for an overhydration of the patient in order to get a corrected Hb mass or concentration value. It also comprises a determination means configured for determining the dose of the agent to be administered starting from or based on the corrected haemoglobin mass or concentration value.

Accordingly, in another aspect of the disclosure a method for determining whether a patient is affected by circumstances leading to the loss of haemoglobin either by loss, bleeding, non-physiological degradation or on any non-physiological account is proposed. The method comprises the step of comparing a concentration or the mass of haemoglobin or an approximation thereof, respectively, measured in a body fluid of a patient and/or an extracorporeal sample thereof at a second point of time with a concentration predicted for the second point of time by means of the method according to any one of claims 1 to 3.

Accordingly, in another aspect of the disclosure the device for determining whether a patient is effected by circumstances leading to the loss of haemoglobin either by loss, bleeding, non-physiological degradation or on any non-physiological account comprises a comparison means configured for comparing a concentration or the mass of haemoglobin an approximation thereof, respectively, measured in a body fluid and/or an extracorporeal sample thereof of a patient at a second point of time with a concentration predicted for the second point of time by means of the method according to any one of claims 1 to 3.

According to yet another aspect to the disclosure, an erythropoesis stimulating agent or medicament expressly intended for use in the treatment of anemia or for enhancing haemoglobin concentration in a patient's blood. It features that the dose and/or the administration scheme or prescription scheme of the medicament is calculated. This medicament could be EPO or iron - also the combined prescription/dosing of at least two different medicaments could be interesting. The patient can be either a human being or an animal. The patient may be sound or ill.

The patient may be in need of medical care or not.

The digital storage means according to the invention is defined by the feature combination of claim 7. Accordingly, in another aspect of the invention, the digital storage means, in particular a disc, CD, or DVD, has electrically readable control signals adapted to interact with a programmable computer system such that a method according to the invention will be executed.

Accordingly, in another aspect of the disclosure the computer program product has a program code stored on a machine readable data medium for executing a method according to the invention when executing the program product on a computer.

Accordingly, in another aspect of the diclosure, the computer program has a program code for the execution of a method according to the invention when executing the program on a computer.

Embodiments according to the invention can include one or more of the following features.

In certain embodiments according to the disclosure, a target or target range for assessing haemoglobin mass or concentration values is created based on haemoglobin mass or concentration values corrected for an overhydration of the patient.

In some embodiments according to the invention, the haemoglobin mass or concentration predicted at the second point is a value corrected for overhydration. In certain embodiments, is it a non-corrected value.

In some embodiments according to the invention, at least one factor is mathematically contemplated or mathematically considered, selected from a group comprising at least:

- an endogenous ESA production;
- a residual renal function;
- a transferrin saturation;

- an administration mode of how EPO or ESA has been or is to be administered;
- a rate of erythrocytes produced from pro-erythroblasts present;
- a factor indicative of the existence and/or degree of physiological endogenous EPO or ESA control;
- a factor indicative of an iron absorption process;
- a factor indicative of the kinetics of various types of exogenous EPO or ESA;
- a factor reflecting the overhydration of the patient;
- a factor accounting for a dosis schedule;
- a factor accounting for or reflecting ferritin and/or hepsidin; and
- a factor describing functional or absolute iron deficiency.

In certain embodiments of the disclosure some or all of the factors noted above have to be expressly stated upon using the method or device according to the present invention. For example, a value may be provided given by the user of the methods or the devices according to the present invention for the factors in questions. In some disclosure, some of the factors may be estimated. In certain disclosure, some of the factors may be replaced by default values. In that case, no value has to be set or provided for by the user for the factor in question; rather, values known from literature or from a look-up reference source or the like may be used instead.

With regard to the present disclosure, ESA is any exogenously administered agent that may be used in the treatment of anemia - it might be EPO or, e. g., iron or the like.
In some embodiments according to the invention, the device comprises means for creating a target or target range for assessing haemoglobin mass or concentration values, the target or target range being is created based on haemoglobin mass or concentration values corrected for an overhydration of the patient.
In certain embodiments according to the invention, the device comprises means for mathematically contemplating or mathematically considering at least one factor selected from a group comprising at least:

- an endogenous ESA production;
- a residual renal function;
- a transferrin saturation;
- an administration mode of how EPO or ESA has been or is to be administered;
- a rate of erythrocytes produced from pro-erythroblasts present;
- a factor indicative of the existence and/or degree of physiological endogenous EPO or ESA control;
- a factor indicative of an iron absorption process;
- a factor indicative of the kinetics of various types of exogenous EPO or ESA;
- a factor reflecting the overhydration of the patient;
- a factor accounting for a dosis schedule; and
- a factor accounting for or reflecting ferritin and/or hepsidin; and
- a factor describing functional or absolute iron deficiency.

In some embodiments of the present disclosure, the iron deficiency can be predicted as follows: a) the lean is measured; b) the blood volume is estimated; the iron dose that is missing is calculated based on, e. g., the following algorithm:

$$\text{Total iron deficiency [mg]} = \text{body weight [kg]} \times (\text{target Hb} - \text{actual Hb}) \text{ [g/l]} \times 0.24 + \text{depot iron [mg];}$$

In above algorithm, the factor 0.24 = 0.0034 x 0.07 y 1000 (iron content of haemoglobin) = 0.34%; the blood volume equals approximately 7% of the body weight; the factor 1000 is used for converting from g to mg. Usually, for people with less than 35 kg body weight, the target Hb is set to 130 g/l, the depot iron is 15 mg/kg body weight; for people with 35 kg body weight and above, the target Hb is set to 150 g/l, the depot iron is 500 mg. Further, in above algorithm, both the actual and the target Hb can be the normohydrated Hb concentration value. For estimating the blood volume, the following algorithm can be used: BVo = 0.1 x LTM + 0.01 x ATM, with LTM referring to lean tissue mass and ATM to adipose tissue mass.

In certain embodiments of the present disclosure, correcting a measured haemoglobin mass or concentration value or an approximation thereof, respectively, for an overhydration of the patient in order to get a corrected mass or concentration value means or comprises a - mere or not - assessing of the measured haemoglobin mass or concentration or an approximation thereof (measured at the first or any other point of time, such as a third one), respectively, in the light of an overhydration.

In some embodiments, the devices according to the disclosure further include means for detecting functional iron deficiency on the basis of the evaluation of the measured/corrected Hb and HCT, and/or means for the detection and quantification of iron deficiency on the basis of a corrected calculation and/or means for the evaluation of TSAT, also referred to as $T_{sat}$, hypochromatic red cells, Hb and the like.

In certain embodiments of the present disclosure, the method comprises one or more steps for detecting functional iron deficiency on the basis of the evaluation of the measured/corrected Hb and HCT, and/or for the detection and quantification of iron deficiency on the basis of a corrected calculation and/or for the evaluation of TSAT, hypochromatic red cells, Hb and the like.

In some embodiments of the present disclosure, predicting of a value can also be understood as approximating, interpolating, extrapolating, or calculating the value.

In certain embodiments of the present disclosure, pharmacokinetic principles are applied.

In certain embodiments of the present disclosure the target Hb concentration is calculated or determined based also on the expected effect of the administered dose of the agent.

In some embodiments of the present disclosure, the erythropoesis stimulating agent is erythropoietin (EPO) or comprises same. In certain embodiments, the erythropoesis stimulating agent is iron (Fe).

In some embodiments of the present disclosure the body fluid is blood. In case of a blood sample, in certain embodiments, the blood sample has been taken from an extracorporeal blood circuit, in other embodiments from a blood vessel of the patient. In some embodiments, the sample is a urine sample.

In some embodiments of the present disclosure, determining the dose means approximating or calculating it.

In certain disclosures the concentration or the mass of haemoglobin is measured, e.g., in blood samples or by means of optical methods, e. g., without having drawn blood from a vessel as it is known in the art. In addition, or alternatively, the values at issue may be derived from other values, parameters, etc. which allow a correct calculation or at least a sufficient approximation of haemoglobin (Hb) or the haemoglobin (Hb) state.

In certain embodiments, the overhydration (OH) is approximated, calculated or defined based on measured values and/or calculations reflecting the overhydration (OH) or the relative overhydration (relOH: overhydration (OH) over extracellular water (ECW)), etc. of the patient. As regards a definition of overhydration (OH) it is referred to WO 2006/002685 A1 where OH equals a*ECW + b*ICW + c*body weight. It is to be understood that OH can be determined in different ways, all of which are known to the person skilled in the art. One of those methods comprises measuring of a dilution and calculate OH based thereon.

In some embodiments, for considering an overhydration, pre-dialysis (pre-Dx) values or calculations are data obtained immediately, i. e., moments or minutes, before starting the next dialysis treatment. The invention is, however, not limited to this. Data can also be obtained at any other point of time. Pre-Dx data appear to be more stable than others. Using them can therefore be of advantage.

In certain embodiments, a target or a target range is defined by means of one threshold or a combination of more than one threshold.

For determining the hydration state or the overhydration any appropriate monitor can be used, such as monitors based on bioimpedance or dilution techniques.

The monitor for obtaining data related to the hydration state can be a monitor as described in WO 2006/002685 A1. Of course, the present invention must not be understood to be limited to monitors determining the hydration state of the patient by bioimpedance measurements as is described in WO 2006/002685 A1. Other methods known in the art such as dilution measurements and also any other method known to the skilled person are also contemplated and encompassed by the present invention as well.

In certain embodiments, the apparatus comprises a monitor for measuring Hb concentrations (e. g., in [g/dl]) and/or for determining the blood volume by means of any monitor as described in *"Replacement of Renal Function by Dialysis"* by Drukker, Parson and Maher, Kluwer Academic Publisher, 5th edition, 2004, Dordrecht, The Netherlands, on pages 397 to 401 ("Hemodialysis machines and monitors").

In some embodiments, the monitor is configured to measure the blood volume and/or the concentration of Hb by means of measuring an electrical conductivity.

In certain embodiments, the monitor is configured to measure the blood volume and/or the concentration of the Hb by means of measuring an optical density.

In some embodiments, HCT and Hb can be measured independently to detect functional iron deficiency.

In certain embodiments, a combination of lab results (e. g., percentage of hypochromatic red cells, TSAT, Hb, HCT, hepsidin ...) can be used to detect functional iron deficiency or iron deficit.

In some embodiments, the monitor is configured to measure the blood volume and/or the concentration of Hb by means of measuring a viscosity.

In certain embodiments, the monitor is configured to measure the blood volume and/or the concentration of the Hb by means of measuring a density.

In some embodiments, the monitor comprises one or more corresponding probes and/or one or more sensors for carrying out the measurements such as electrical conductivity sensors, optical sensors, viscosity sensors, density sensors, and the like.

In certain embodiments, the device may be used also for treating a patient by means of dialysis.

In other embodiments, the device may be used for treating a patient (or the patient's blood) by haemofiltration, ultrafiltration, haemodialysis, etc.

Additional embodiments according to the present invention are defined by the feature combinations of the claims attached hereto. For avoiding repetition, these feature combinations are made part of the present specification by way of reference. The embodiments may provide one or more of the following advantages.

In certain embodiments according to the disclosure, the effects of the changing fluid status from the (frequently performed) Hb measurements (e. g., calculating the normohydrated Hb) can be eliminated.

In some embodiments according to the disclosure, a predefined Hb target range is correctly reached.

In certain embodiments according to the disclosure, a predictive control of Hb in a given target range is achieved.

In some embodiments according to the disclosure, a variability (fluctuations) of Hb are prevented.

In certain embodiments according to the disclosure, it is possible to predict the steady state Hb plateau (steady state Hb with a given and constant EPO dose).

In some embodiments according to the disclosure, changes in the Hb degradation, Hb losses, e. g., through bleeding, haemolysis and the like become predictable.

In certain embodiments according to the invention it allows to predict in advance when the patient will reach a steady state in the target range.

In some embodiments according to the disclosure, Pre knowledge of the system dynamics allows to interact timely with the ESA administration

In certain embodiments according to the disclosure a system is provided to achieve Hb stability over time in a predictive way, by taking into account the underlying physiological processes.

In some embodiments according to the disclosure for the first time a system or model combining the effects of fluid status, iron status, ESA (EPO, in particular) administration, EPO and/or iron administration and Hb kinetics is proposed.

In certain embodiments according to the disclosure, a closed ESA or EPO and Hb loop is modeled.

In some embodiments according to the disclosure a combination of the effects of both iron and EPO and possible interactions is proposed. Hence, effects on Hb due to the iron balance of the patient's body may be considered upon EPO administration.

In some embodiments according to the disclosure, a steady state Hb plateau can advantageously be predicted.

In certain embodiments according to the disclosure, a ESA dose required to achieve targeted Hb can advantageously be predicted.

In some embodiments according to the disclosure, Hb variability can advantageously be eliminated.

In certain embodiments according to the disclosure, unphysiologic changes in the Hb mass can advantageously be predicted. Thus, events such as internal bleeding, blood loss, coagulation, infections, and the like can advantageously be detected.

In some embodiments according to the disclosure, kinetics of various ESA or EPO types may be included. Hence, features such as different half-lives can be accounted for.

In certain embodiments according to the invention, dosing schedules (weekly, daily, monthly...) may be taken into account.

In some embodiments according to the disclosure, an optimal dosage of iron and, in consequence, EPO can be found based on the understanding that TSAT may be the limiting factor for the uptake of iron or ferritin into the cells. Assessing the transferrin saturation may prevent from overdosing iron. Since iron may contribute to inflammatory processes within the patient's body, by applying or calculating an adequate dosage of iron according to the principles of the present invention, adverse overdosing of iron may be advantageously be prevented. The transferrin saturation can easily be assessed by the number of hypochromatic red cells (red cells with a low content of haemoglobin).

Also, according to the present disclosure, a time scheme may be proposed in which iron and EPO are applied at different points of time, taking the particular interaction of EPO (influencing the number of red blood cells generated) and iron (responsible for the quality of the red cells with regard to oxygen-binding capacity) advantageously into account.

Other aspects, features, and advantages will be apparent from the description, figures, and claims.

Fig. 1    shows a first apparatus comprising a controller for carrying out the method according to the invention;

Fig. 2    shows a second apparatus comprising a controller for carrying out the method according to the invention;

Fig. 3    shows the concept of two reference ranges;

Fig. 4    shows the concept of Hb regulation in health;

Fig. 5    shows the regulation of Hb with partial renal function (RF) or zero renal function;

Fig. 6    represents the fractional mass per gram of erythrocytes over the erythrocytes lifetime in days;

Fig. 7    shows the accumulation of Hb mass due to each new pulse of erythrocytes generated daily;

**Fig. 8**       represents the mass of Hb in circulation using an array of FIFO buffers;
**Fig. 9**       illustrating the erythroblast conversion over the transferrin saturation;
**Fig. 10**      reveals a pro-erythroblast transfer function;
**Fig. 11**      shows an expanded section of EPO to pro-erythroblast transfer function of Fig. 10;
**Fig. 12**      shows the gain of EPO as a function of the renal function;
**Fig. 13**      shows the derivation of the concentration of EPO appearing in the vascular space in a scheme;
**Fig. 14**      shows a scheme for intravenous infusion of EPO; and
**Fig. 15**      shows the renal function over the renal creatinin clearance.

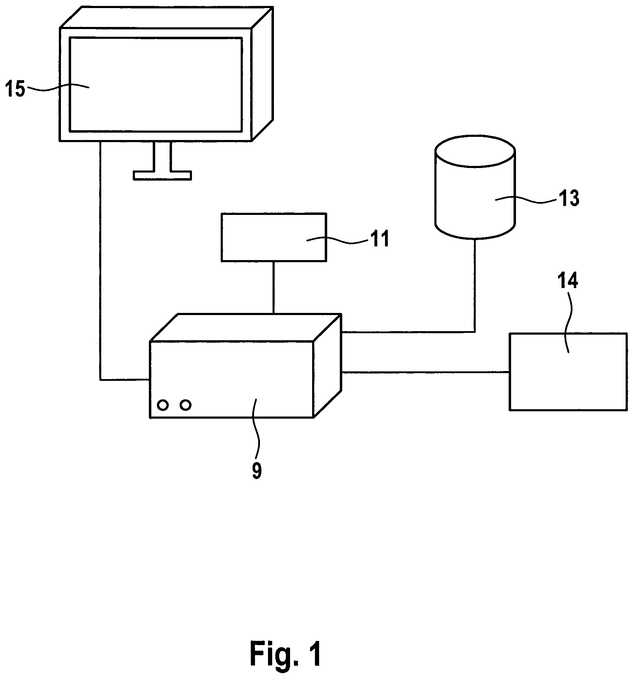

The present invention is defined according to the appended claims. **Fig. 1** shows an apparatus 9 comprising a controller 11 for carrying out the method according to the invention. The apparatus 9 is connected to an external database 13 comprising the results of measurements and the data needed for the method according to the invention. The database 13 can also be an internal means. The apparatus 9 may optionally have means 14 for inputting data into the controller 11 or into the apparatus 9. Such data may be information about the mass, the volume, the concentration of Hb as is set forth above. Such data input into the apparatus 9 may - additionally or instead - also be information about the overhydration of the patient or an approximation thereof. The results of the prediction, evaluation, calculation, comparison, assessment etc. performed by the controller 11 and/or the apparatus 9 can be displayed on the monitor 15 or plotted by means of a - not displayed but optionally also encompassed - plotter or stored by means of the database 13 or any other storage means. The database 13 can also comprise a computer program initiating the method according to the invention when executed.

In particular, the controller 11 can be configured for carrying out any method according to the invention.

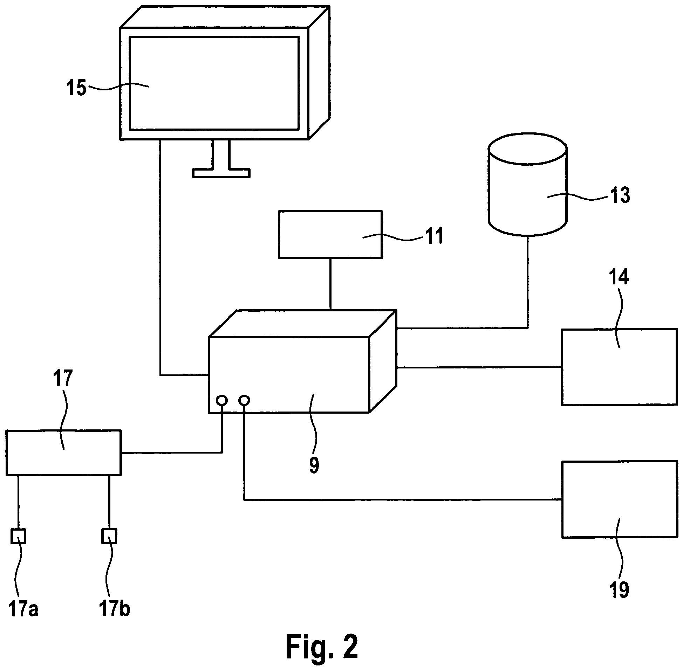

As can be seen from **Fig. 2,** for corresponding measurements, the apparatus 9 can be connected (by means of wires or wireless) with a bioimpedance measurement means 17 as one example of a means for measuring or calculating the hydration state or an overhydration state. Generally, the means for measuring or calculating the hydration state or an overhydration state can be provided in addition to the external database 13 comprising the results of measurements and the data needed for the method according to the invention, or in place of the external database 13 (that is, as an substitute).

The bioimpedance measurement means 17 can be capable of automatically compensating for influences on the impedance data like contact resistances.

An example for such a bioimpedance measurement means 17 is a device from Xitron Technologies, distributed under the trademark Hydra™ that is further described in WO 92/191 53.

The bioimpedance measurement means 17 may comprise various electrodes. In Fig. 2, only two electrodes 17a and 17b are shown which are attached to the bioimpedance measurement means 17. Additional electrodes are, of course, also contemplated.

Each electrode implied can comprise two or more ("sub"-)electrodes in turn. Electrodes can comprise a current injection ("sub-")electrode and a voltage measurement ("sub")electrode. That is, the electrodes 17a and 17b shown in Fig. 2 can comprise two injection electrodes and two voltage measurement electrodes (i. e., four electrodes in total).

Generally spoken, the means for measuring or calculating the hydration state or an overhydration state can be provided by means of weighing means, a keyboard, a touch screen etc. for inputting the required data, sensors, interconnections or communication links with a lab, any other input means, etc.

Similarly, the apparatus 9 may have means 19 for measuring or calculating means for obtaining a value reflecting the mass, the volume or the concentration of the substance that can again be provided in addition to the external database 13 already comprising the results of measurements and the data needed for the method according to the invention, or in place of the external database 13 (that is, as an substitute).

The means 19 can be provided as a weighing means, a keyboard, touch screen etc. for inputting the required data, sensors, interconnections or communication links with a lab, a Hb concentration probe, any other input means, etc.

Again, it is noted that the figures relate examples showing how one embodiment according to the invention may be carried out. They are not to be understood as limiting.

Also, the embodiments according to the invention may comprise one or more features as set forth below which may be combined with any feature disclosed somewhere else in the present specification wherever such combination is technically possible.

In the following, three different ways to achieve the target of the invention are described in detail. They should, however, not be understood as intended to limit the invention in any way.

A **first** embodiment of the invention is called the straight forward approach and will be explained in the following without making reference to any figure. The straight forward approach comprises or consists of the following steps, findings or considerations:

In the straight forward approach, if one or more pre-measured (i. e., measured before carrying out the method according to the present invention) concentration values or mass values of haemoglobin (short also: Hb) are found to be below the target range suggested by guidelines or requested by the physician in charge for a particular patient based on prior art knowledge, the prescription of the patient's dose of ESA (or EPO, in particular) is stepwise increased, particularly in form of a mental or academic act, e. g., in form of hints regarding to the prescription of EPO.

Also, Hb concentration or mass values obtained by frequent measurements (in certain embodiments these are values that had been obtained before and/or after every dialysis treatment, that is, not as part of the present method) are considered in the present straight forward approach.

Further, values reflecting the patient's fluid overload - which in particular have been obtained from measuring on a irregular or regular basis prior to carrying out the present method - are considered. The values reflecting the patient's fluid overload may have been obtained at the moment in which also the Hb concentration or mass values have been obtained.

In another step, some or all of the measured Hb values are computationally or mathematically corrected for the fluid overload to obtain the normohydrated Hb levels.

Also, in yet another step, measures are requested or contemplated at least for correcting the fluid status as another mental or academic step - it may at least be intended to correct the fluid status to levels of, e. g., TAFO (time averaged fluid overload, see also below) = 0.3-0.5 litre (L). It is noted that a step of correcting the fluid status by therapy is in certain embodiments of the invention not part of the method according to the invention, whereas in others it may be. A correction of the fluid status may, of course, be contemplated or take place independently from the method described in here. TAFO, the time averaged fluid overload, means the time averaged fluid overload indicating the average fluid status over one week - thus compensating for the peak overhydration before the haemodialysis session and the possible dehydration after the haemodialysis session. The TAFO can also be used when haemodialysis and peritoneal dialysis patients are compared - the fluid status measured in peritoneal dialysis patients is very close to the TAFO. Studies have shown that a TAFO of 0.3-0.5 L could be achieved.

A highly suitable time for carrying out this correction may in certain embodiments be in the order of the time lag between EPO dose change and expected start of change in the Hb mass. Usually, this time lag is about 20 days. Hence, in some embodiments, the correction is carried out about 20 days after the last administration of exogenous EPO. EPO has to be understood as one example of a erythropoesis stimulating agent only.

A curve (a line or a 1 st order function) is in some embodiments fitted in a suitable diagram (e. g., normohydrated Hb over time) through the normohydrated Hb values and extrapolated.

In any way, the straight forward approach may finally comprise a check if and when this extrapolation will meet the prior art Hb target or target range. The slope of the Hb rise will depend on the EPO concentration. The life time of the erythrocytes will determine which steady state Hb level will be reached. In this respect, it is noted that certain diseases or physical states can influence the red blood cell (RBC) lifetime in a negative way - e. g., acute inflammatory situations can decrease the RBC lifetime significantly, as is stated in literature. In generally healthy subjects, the average lifetime of RBC can be assumed to be constant around 120 days. Chronic kidney disease (CKD) patients might however have shorter RBC lifetimes.

One of the advantages provided by the straight forward approach according to the invention is that is does not necessarily need a sophisticated model accounting for the EPO or Hb kinetics. Hence, this approach is easy to handle and does neither need particular effort nor computing resources.

A device intended for carrying out the method described above with regard to the straight forward approach will comprise means for at least each step to be carried out.

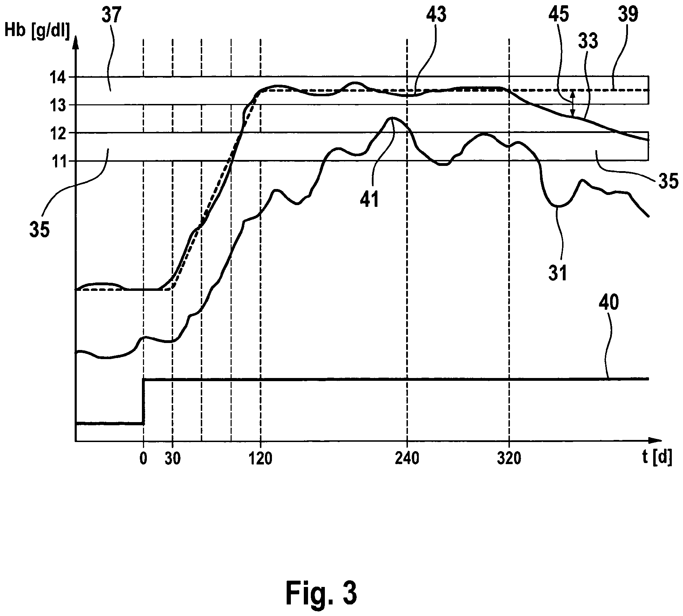

With respect to Fig. 3, a **second** embodiment of the invention, called a simplified Hb control based on normohydrated Hb, will be explained in the following.

A major feature of this second embodiment is defining a target range based on the normohydrated Hb. In the following, this target range is called a second target range irrespective of whether there is also a first target range or not so as to emphasize that this second target range may be different from a commonly observed - and therefore referred to as "first" - Hb target range proposed by, e. g., guidelines.

The second target range of the normohydrated Hb will in most cases be higher than (first) guideline values for Hb measured - the guidelines refer to a typical predialytic situation to set up the target, a situation in which the patient is ca. 2 L fluid overloaded.

In the second embodiment or model, the Hb concentration is measured on a frequent basis. The fluid status is also measured on a frequent basis. In certain embodiments, "frequent" refers to, e. g., once weekly or even at every treatment - in acute patients the measurement frequency could be several times per day.

In accordance with the present invention, the control of Hb is based on the second reference range (Hb normohydrated).

The second reference range could also allow comparing different dialysis centres with different fluid stati for reasons of quality controls. Currently, it is difficult to compare the Hb concentrations between treatment centers - most centers have currently different policies towards the fluid management, and the measured Hb concentrations (found before or after dialysis) will be influenced by this effect. If, however, normohydrated Hb values are compared between the centers, the fluid effect is already compensated; therefore, the comparison is much easier.

Fig. 3 reveals the concept of having two reference ranges - one for the normohydrated Hb, one for the Hb concentration range as suggested by presently observed guidelines, or to have even only one reference range, the one for the normohydrated Hb.

As can be seen from Fig. 3, which shows the development of the concentration of Hb [g/dl] of a particular dialysis patient over time t [in days (d)]. A measured Hb curve 31 (solid curve) depicts the measured Hb concentration over time. A normohydrated Hb curve 33 (bold curve) depicts the measured Hb concentration after having the Hb concentration values corrected for fluid overload (therefore, curve 33 is called "normohydrated").

Fig. 3 further reveals a first target range 35, also called the target range for Hb as measured. It may be set according to guidelines presently considered. In Fig. 3, the first target range 35 is only shown for illustrating the differences and advantages of the model according to the present embodiment of the invention when compared with the prior art methods. Fig. 3 reveals also a second target range 37 as proposed by means of the present invention for the corrected Hb concentration or the normohydrated Hb, respectively, see curve 33.

As can be seen from Fig. 3, a simplified model prediction, expressed by a dotted model prediction curve 39 is assessed by means of second target range 37 provided for the predicted Hb concentration values.

In Fig. 3, the increased dose 40 of ESA, applied on day 0 has lead to an increase both of measured Hb curve 31 and normohydrated Hb curve 33.

As can further be seen from Fig. 3, the corrected or "normohydrated Hb" curve 33 is much smoother when compared to measured Hb curve 31. Hence, the temptation for the physician to amend the ESA dose without need due to given fluctuations of the Hb concentration such as to be seen at reference numeral 41 depicting a maximum Hb value measured on day 240 is much lower when the normohydrated Hb curve 33 and its position within the second target range is considered instead of measured Hb curve 31 and its corresponding first target range 35.

As can further be seen from Fig. 3, also due to the smoother characteristic of normohydrated Hb curve 33, when compared to measured Hb curve 31, events such as the occurrence of blood loss due to, e. g., bleeding, the onset of which is depicted at reference numeral 43 on day 320, are earlier discovered as is readily understood by means of Fig. 3.

In Fig. 3, arrow 45 shows a difference between the model prediction curve 39 and the measured normohydrated Hb curve 33. As was explained above, in the particular example of Fig. 3, this difference or deviation finds its reason in an internal bleeding.

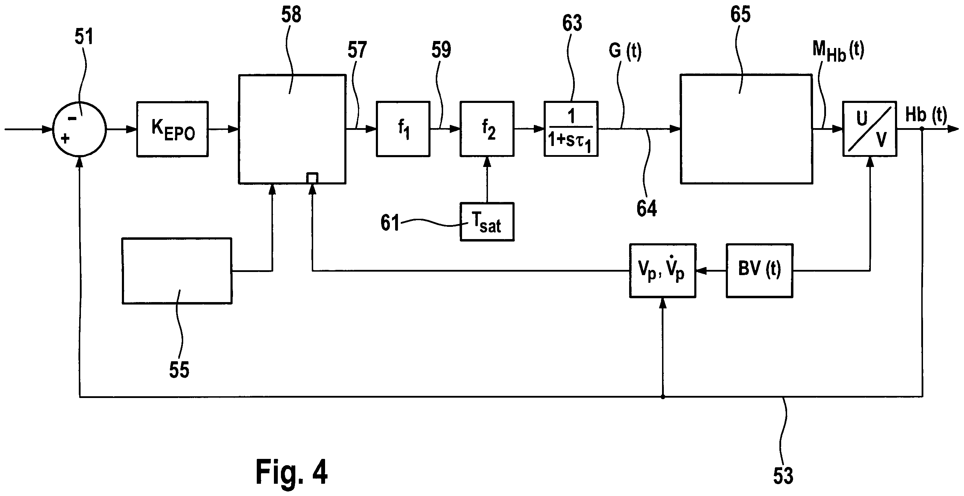

A **third** embodiment of the invention is called by the inventors a Hb kinetic model and will be explained in the following with respect to the Figs. 4 to 14.

The general idea of the model may be described as follows:

In health, receptors that monitor oxygen delivery control the secretion of erythropoietin (also referred to as EPO being an <u>e</u>rythrocyte <u>s</u>timulating <u>a</u>gent, ESA) that regulates the production of pro-erythroblasts from colony forming units. Depending on the level of available iron (e. g., measured with the TSAT level) the content of heme or haemoglobin in the red blood cells (RBC) is influenced. TSAT has no impact on the number of RBC; rather, it is needed to fully equip the RBC with haemoglobin. Low TSAT and high ferritin levels are, e. g., a marker of possible iron deficiency. The supply and destruction of erythrocytes ultimately determines the Hb mass that is maintained in a subject. Normally, when Hb levels decrease this causes an increase in EPO concentration to stimulate more production of erythrocytes.

**Fig. 4** shows the concept of Hb regulation in health. The underlying model may be explained as follows:

As can be seen from the operator depicted at reference numeral 51, the Hb regulation in health is controlled also by means of a feedback loop 53.

The production of erythrocytes is stimulated from a baseline endogenous EPO production 55 only. The total intravasal or intravascular, respectively, EPO concentration (or the part hereof that can be measured from, e. g., venous blood samples) is indicated by reference numeral 57. The EPO concentration is influenced by EPO kinetics, indicated by reference numeral 58.

Depending on a function f1 describing the production of pro-erythroblasts from a given EPO concentration, pro-erythroblasts 59 are produced/generated in the model in question.

A given saturation 61 of transferrin (TSAT) in the body has a certain influence via another function f2 on the generation rate $G^*_{Hb}(t)$ of haemoglobin 64 produced. TSAT will not influence the number of Hb containing cells (HCT) but the heme (haemoglobin) content of these cells. This effect is modeled by a quality indicator Q, which is dependent on TSAT.

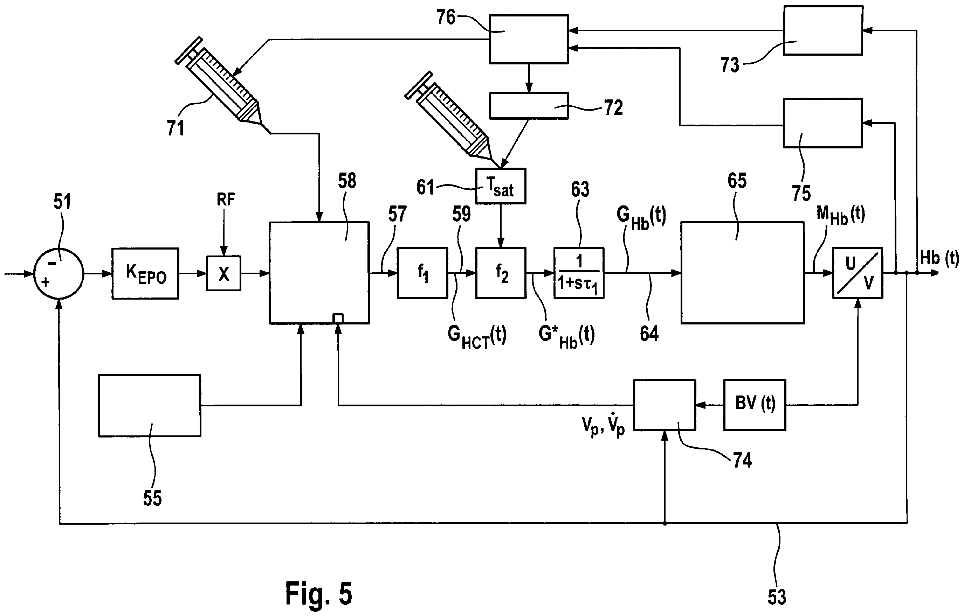

A finally resulting Hb mass, indicated as $M_{Hb(t)}$ is also influenced by erythrocyte kinetics 65. The ratio u/v allows calculating the corresponding Hb concentration Hb(t) and vice versa. Vp is calculated because the EPO is distributed in the plasma volume. Vp is influenced by the fluid status (hydration) - the rate of change of Vp is thus relevant for the EPO dosing - e. g., if Vp goes down, the concentration of EPO will go up - see also equation 34 and Fig. 5.

A number of the items referred to with regard to Fig. 4 is explained below in more detail.

In contrast, in case of disease there may be only partial renal function and some of the ability to regulate Hb physiologically is lost ($K_{EPO}$ is reduced), see **Fig. 5** showing the regulation of Hb with partial renal function (RF) or zero renal function, requiring administration of exogenous EPO. With zero renal function there is no physiological regulation of Hb because the feedback loop is hampered. In either case, EPO levels usually need to be supplemented with exogenous EPO 71 and/or with iron 72, which is, e. g., IV administered. The feedback loop 53 then has to be re-established or substituted, e. g., by measuring Hb on a monthly basis in the clinic, e. g., by means of assessing a blood sample, and by adjusting the exogenous or administered EPO dose accordingly. Plasma kinetics are depicted at 74, BV(t) indicates the blood source.

Now with the advent of technology such as the means 73 for monitoring the body volume of the patient and means 75 for measuring the Hb concentration or mass comprised by the patient's body, the possibility exists for more frequent monitoring. The means 73 for monitoring the body volume could be used fortnightly, for example, to obtain volume information. The means 75 for measuring the Hb concentration can easily monitor Hb at every treatment. Such means are well known to the skilled person. Examples of means suitable for the purposes discussed in this paragraph are disclosed, for example, in Fig. 1 and Fig. 2.

Using the means 73 and 75 as feedback sensors contributes to determining the exogenous EPO and/or iron level that both allows the target Hb to be achieved whilst maintaining reasonable Hb stability by means of ESA control 76 (with ESA referring to EPO and iron in the example of Fig. 5).

In this model according to the third embodiment according to the invention, the erythrocyte lifetime and Hb mass may be determined as is explained below:

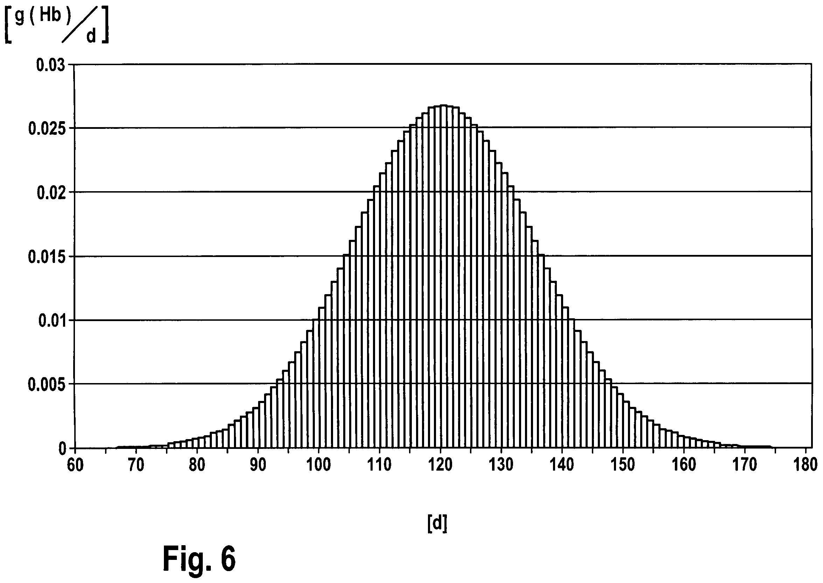

Erythrocytes have a lifetime of typically 120 days before being destroyed and much of the constituent components being recycled. The lifetime of erythrocytes is normally distributed as shown in **Fig. 6** representing the fractional mass per gram of erythrocytes being eliminated from the patient's circuit or body. As can be seen from Fig. 6, the first RBC produced on day "0" are eliminated on day 67, the last ones on day 174; the mean lifetime is 120 days. Knowing the typical lifetime span may allow in certain embodiments to calculate the production rate (if Hb is known). This applies at least for the steady state.

In order to grasp how the variable lifetime affects the accumulation of haemoglobin mass, it is convenient to consider a discrete production process, even though the production of erythrocytes is continuous.

Each day, a "pulse" of erythrocytes are generated, represented by the daily generation rate $G_{Hb(t)}$. Within this pulse, sub units of erythrocytes are generated with specific lifetimes such that

$$G_{Hb}(t) = \sum_{i=1}^{N} g_i(t) \qquad\qquad \text{Eq 1}$$

, where N is the number of lifetime elements considered in the distribution and i is the lifetime index. "i" is a unit of time - it may refer to hours, days, week, and the like. In the case of days, "i" may run from, e. g., 1 to 180.

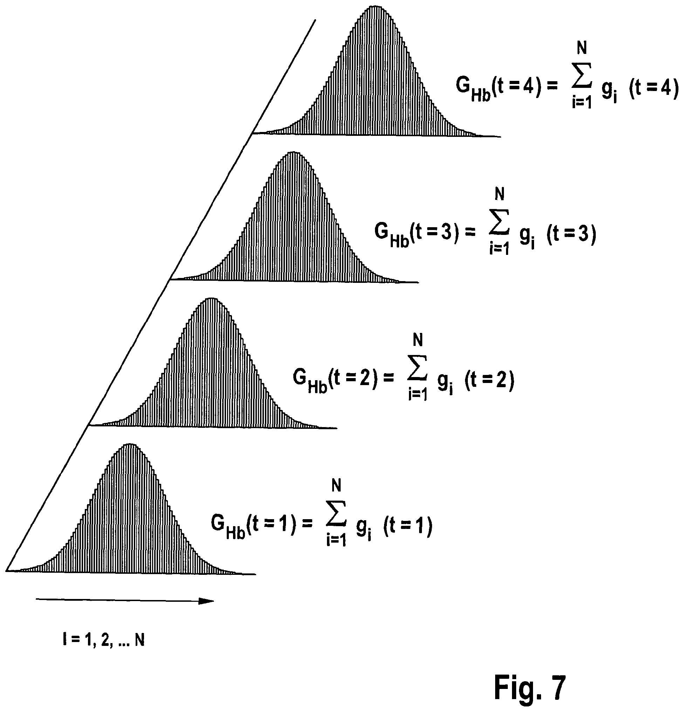

Fig. 7 shows the accumulation of Hb mass due to each new pulse of erythrocytes generated daily. The sum of the sub units with specific lifetimes, $g_i$, under the pulse distribution is referred to as the daily generation rate.

The value of the sub units $g_i$ may be calculated from a Gaussian function:

$$g_i(t) = \frac{G_{Hb}(t)}{\sqrt{2\pi\sigma^2}} \cdot e^{\frac{-(eLT_i - \mu)^2}{2\sigma^2}} \qquad\qquad \text{Eq 2}$$

where $\mu$ is the mean erythrocyte lifetime and $\sigma$ is the standard deviation of the distribution and therefore the erythrocyte lifetime (eLT) has the range

$$\mu - 3\sigma \leq eLT \leq \mu + 3\sigma \qquad\qquad \text{Eq 3}$$

Taking three standard deviations of the mean lifetime allows 99.7% of the distribution to be considered whilst allowing reducing computational overhead. Setting $\mu$ = 120 days and $\sigma$ = 15 days gives a typical distribution.

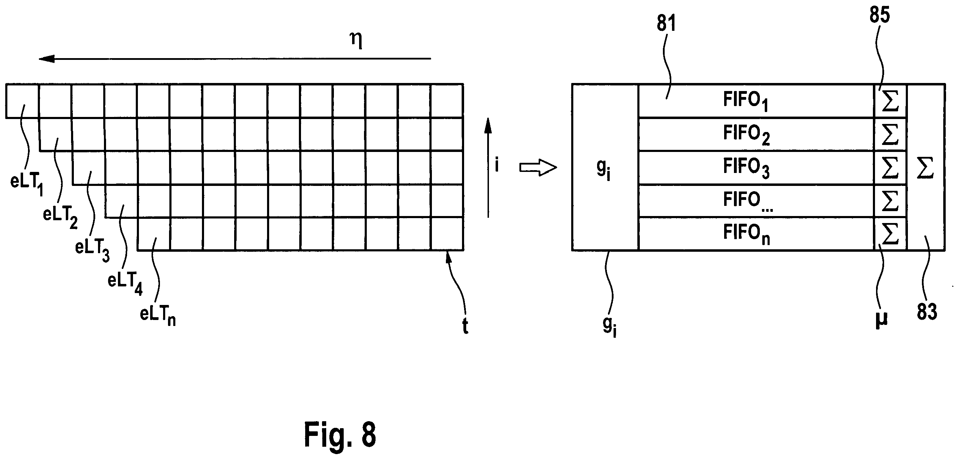

The mass of Hb, $M_{Hb(t)}$, can be calculated by considering an array of FIFO (First-In-First-Out) buffers, the length of each buffer representing a specific erythrocyte lifetime, see **Fig. 8** representing the mass of Hb in circulation using an array of FIFO buffers with buffers $FIFO_1$ to $FIFO_n$ forming a FIFO memory 81. In Fig. 8, different erythrocyte lifetimes

are depicted as $eLT_1$ to $eLT_n$. A pulse distributer is represented by $g_i$ and the Hb mass sum 83 is computed on basis of one sum 85 related to each FIFO buffer weighted by $\mu$.

As each pulse of erythrocytes is generated as shown in Fig. 7, the pulse passes through the FIFO buffers. The total mass 83 of Hb in circulation is the sum of all storage elements $FIFO_1$ to $FIFO_n$ in the FIFO buffer.

The total mass 83 is thus:

$$M_{Hb}(t) = \sum_{i=1}^{N}\sum_{\eta=0}^{eLT_i} g_i(t-\eta)\Delta T \quad \forall t > \eta \qquad \text{Eq 4}$$

The mass however changes depending only on the mass entering and leaving the FIFO buffer at a given time instant. Hence the mass is preferably computed even more efficiently as

$$M_{Hb}(t) = \sum_{i=1}^{N} g_i(t)\cdot\Delta T \quad \forall t <= eLT_i \qquad \text{Eq 5}$$

and

$$M_{Hb}(t) = \sum_{i=1}^{N} g_i(t)\cdot \Delta T - g_i(t-eLT_i)\cdot \Delta T \quad \forall t > eLT_i \qquad \text{Eq 6}$$

**Erythrocyte 64 production**

The processes involved in erythrocyte production, which is referred to as being part of the erythrocyte kinetics 65 of Fig. 4 and Fig. 5, encompass three main elements, namely the production of pro-erythroblasts from a given EPO concentration based on function f1 of Figs. 4 and 5, the "loading" of the pro-erythroblasts with heme - influenced by TSAT (f2) - and a production transport delay 63.

In most practical cases, the production rate of erythrocytes 64 can be determined easily from measurements. Also, the production rate may be calculated from the Hb concentration (known), the Gauss distributed life time (also known, e. g., from literature); based on this, the production rate may be calculated in a reverse manner. Once calculated, the production rate can be compared to data known from literature. With regard to the production rate, the model can be adapted to the particular patient.

The present invention contributes to calculating the EPO concentration that is necessary to support the given production rate. Following three processes, shown in Figure 4 and Figure 5 as f1, f2 and the production time constant 63, are of relevance to do so for certain embodiments. Therefore, they are explained in the following in the reverse order, or, with reference to Fig. 4 or 5, from right to left:

**Production time constant 63**

The production of erythrocytes involves several processes from the time when EPO doses changes until a change in the generation rate may be observed (or takes place). According to Guyton, after a change in EPO doses, new erythrocytes do not appear in the circulation for 2 to 4 days and the maximum rate of new production is observed after 5 days or more. This effect may be characterized by a first order lag with a time constant, $\tau$ of the order of 2.5 days. This could be a transport delay, but we are simplifying it with a first order lag.

$$G_{Hb}(t) = \frac{1}{\tau}\int_0^t G{*}_{Hb}(t) - G_{Hb}(t)dt \qquad \text{Eq 7}$$

$G_{Hb}(t)$ represents the generation rate of erythrocytes. $G^{*}_{Hb}(t)$ is used to denote the production rate before the first order lag and $G_{Hb}(t)$ after the first order lag. In other words, $G_{Hb}(t)$ lags behind $G^{*}_{Hb}(t)$ and in steady state $G_{Hb}(t) = G^{*}_{Hb}(t)$.

**Transferrin saturation 61 (TSAT)**

The loading of the pro-erythroblasts 59 with heme depends on the availability of iron - which can be simplified by using TSAT. Thus, TSAT defines the quality of the erythrocytes; it states or accounts for how much haemoglobin is in the RBC. It is assumed that the relation between TSAT and $k_Q$ (being the quality indicator of the RBC) is non-linear requiring some saturation effect. This is modeled with an exponential function as a first proposal. Thus, the generation of Hb, $G_{Hb}(t)$, is related to the generation rate of pro-erythroblasts $G_{HCT}(t)$ via f1 and the loading of heme into the RBC (f2).

$$f_2(t) = G^*_{Hb}(t) = G_{HCT}(t)\left(1 - e^{-kQ}\right) \qquad \text{Eq 8}$$

In the inverse form the generation of pro-erythroblasts is:

$$G_{HCT}(t) = \frac{G^*_{Hb}(t)}{\left(1 - e^{-kQ}\right)} \qquad \text{Eq 9}$$

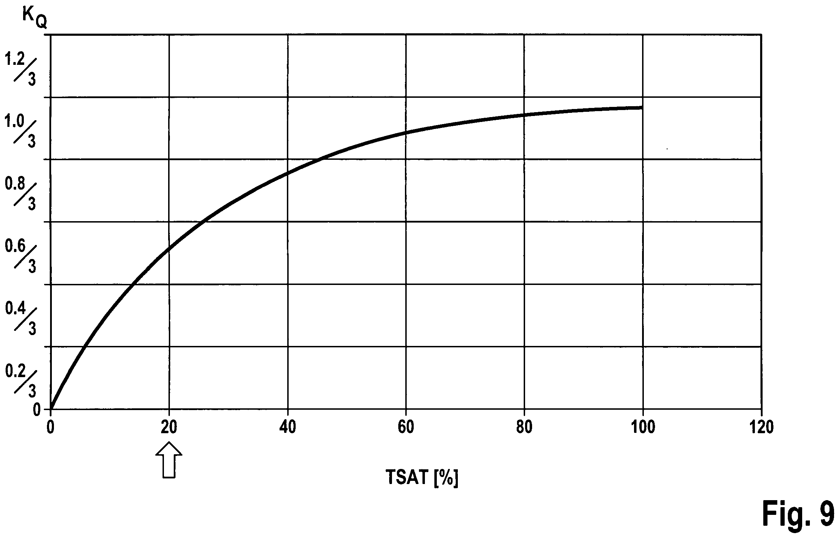

where $k_Q$ is the constant describing the filling of the erythrocytes with heme - depending on the transferrin saturation rate - guidelines (e. g., European best practise guidelines) propose a TSAT > 20% threshold for dialysis patients (see arrow in **Fig. 9**).

**Pro-erythroblast 59 generation**

The rate of pro-erythroblast production depends on the concentration of EPO in the vascular space. For the purposes of derivations that follow, the concentration of vascular EPO will be denoted by [EPOagg]v. The subscript "v" distinguishes vascular (or plasma) concentrations of EPO from subcutaneous concentrations as discussed later regarding the topic of exogenous EPO administration. The subscript "agg" indicates the aggregate concentration of EPO formed from both endogenous and exogenous sources. Based on its origin, below, EPO is denoted respectively as [EPOe]v and [EPOx]v originating from different sources. This differentiation appears helpful because endogenous EPO can have a different half life than exogenous EPO.

To avoid confusion, the following argument is developed with the variable [EPOagg]v, regardless of whether it is solely endogenous EPO or a combination of both endogenous and exogenous EPO. The physiological range of [EPOagg]v within the plasma volume is 10 to 30 [u/L]. In extreme cases, the concentration can increase 100 to 1000 fold in a healthy subject. Whether this translates to a proportional increase in pro-erythroblast production rate seems unlikely as can be seen from the following argument:

Firstly the mass of Hb in circulation in steady state is the product of mean erythrocyte lifetime and production rate and this is also equal to the product of the current Hb and blood volume, i. e.,

$$M_{Hb}(ss) = BV \cdot Hb = G_{Hb} \cdot \mu \qquad \text{Eq 10}$$

with "SS" indicating the "steady state".

Taking a typical blood volume of 50 dl, an Hb of 14 g/dl leads to

$$G_{Hb} = \frac{BV \cdot Hb}{\mu} \qquad = 5.833 \text{ g/day} \qquad \text{Eq 11}$$

In steady state $G_{Hb}$ equals $G_{ery}$. Therefore, if the transferrin saturation TSAT is 20%, then the corresponding generation of pro-erythrocytes from Eq 9 is 11.68 ml/day

A 100 fold increase in $G_{pro}$ to 1.168 kg/day is highly unlikely and a 1000 fold increase in $G_{pro}$ to 11.68 kg/day is completely implausible. This would be the basis therefore for a saturation function, modeled in the simplest form as an exponential of the form:

$$G_{HCT} = G_{HCT\_max} \cdot \left(1 - e^{-k \cdot [EPO_{agg}]_v}\right)$$   Eq 12

Taking the middle of the physiological range is an EPO concentration in the vascular space [EPOagg]v of 20 u/L and assume this can increase 100 fold. Assuming therefore that 2000 u/L leads to 99.99% of the maximum production rate of pro-erythrocytes then

$$1 - 0.9999 = e^{-2000 \times k}$$   Eq 13

From which k = 0.002

$$G_{HCT}\max = \frac{11.68}{1 - e^{-k \times 20}} \approx 298 \text{ ml/day}$$   Eq 14

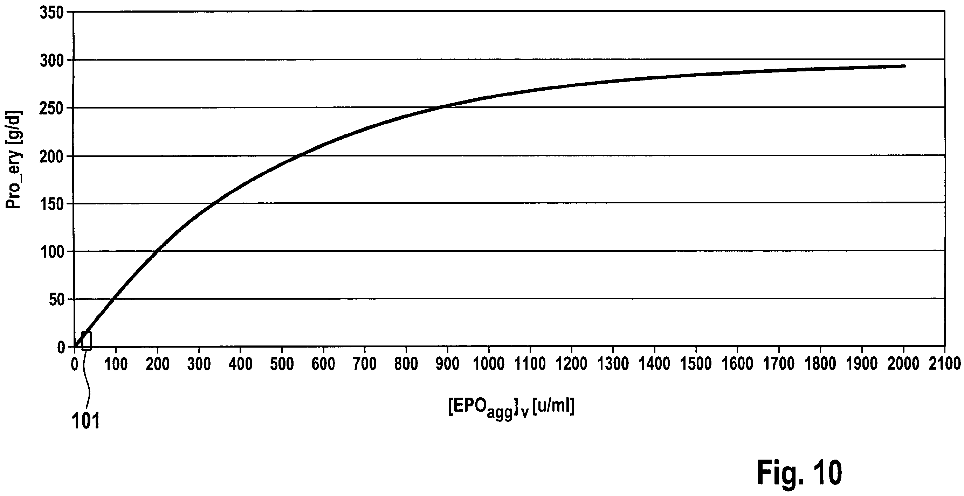

**Fig. 10** reveals a pro-erythroblast transfer function f1, illustrating Pro_ery [g/d, i. e., gram/day] over [EPOagg]v in unit per millilitre [u/ml] ("ml" relates to the blood volume). In Fig. 10, the normal physiological range is referred to by reference numeral 101.
298 ml/day peak production rate might be just plausible, but it should be recalled that this represent an extreme condition. For normal physiological ranges even with significant deviations of 10 fold, the function is largely linear.

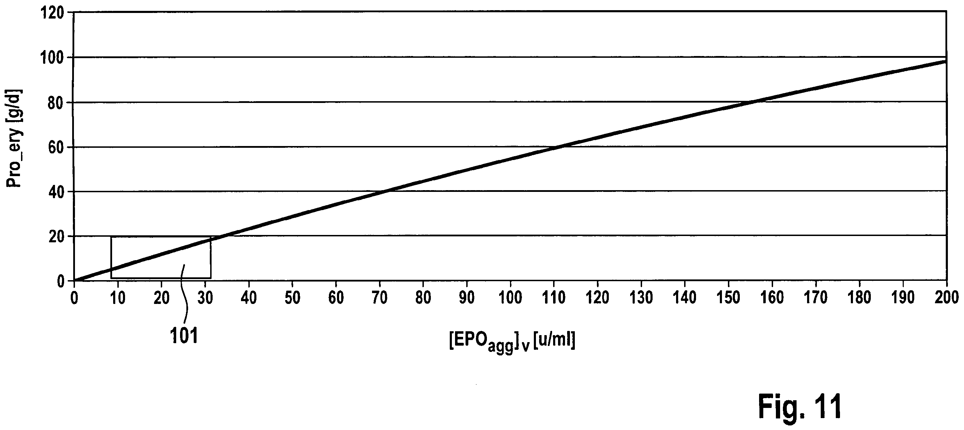

**Fig. 11** shows an expanded section of EPO to pro-erythroblast transfer function f1. The linear function is a good approximation even for a 10 fold increase in EPO.

**Baseline endogenous EPO production 55**

Using the same principles as above, it is possible to estimate the baseline endogenous EPO production rate. In this case, the source of EPO is clear and therefore use of the variable [EPOe]v is appropriate. The easiest way to achieve this is to consider a healthy subject that then develops anemia due to fluid overload. The blood volume is increased by an extra litre from 5 L to 6 L, and the patient's steady state Hb falls to 8 g/dl.
In some embodiments, it is assumed that mean erythrocyte lifetime remains normal at 120 day. In certain embodiments, it is assumed that TSAT is maintained in the healthy range of 40% by suitable iron therapy.
By combining Eq 9 and Eq 11, the production rate of pro-erythroblasts is given by:

$$G_{HCT}(t) = \frac{Hb \cdot BV}{\mu \cdot \left(1 - e^{-KQ}\right)}$$   Eq 15

Substituting above values, the production rate of pro-erythroblasts is 6.66 g/day. Rearranging Eq 12 yields:

$$[EPO_e]_v = \frac{-1}{k} \cdot \ln\left(1 - \frac{G_{HCT}}{G_{HCT\_Max}}\right)$$   Eq 16

Thus, the EPO concentration corresponding to 8 g/day pro-erythroblast generation rate is 13.61 u/L. "L" relates to the plasma volume.
The baseline flux of EPO (generation rate) into the vascular system $F_{EPO\_baseline}$ must balance the flux out of the vascular system caused by liver clearance. The flux out is the product of EPO concentration and liver clearance:

$$F_{EPO\_baseline} = [EPO_e]_v \cdot K_{Liver}$$   Eq 17

From which $F_{EPO\_baseline}$ = 0.0136 x 1.75 = 0.0238 U/min (see later sections for determination of liver clearance). In other words in the absence of physiological regulation, baseline endogenous EPO flux supports a steady state Hb of 8g/dl. This is valid for a subject with a normal blood volume of 50 dL. Subjects with higher and lower BVs will need to be scaled accordingly. Therefore,

$$F_{EPO\_baseline} = \frac{BV}{50} \cdot 0.0238 \quad \text{U/min} \qquad \text{Eq 18}$$

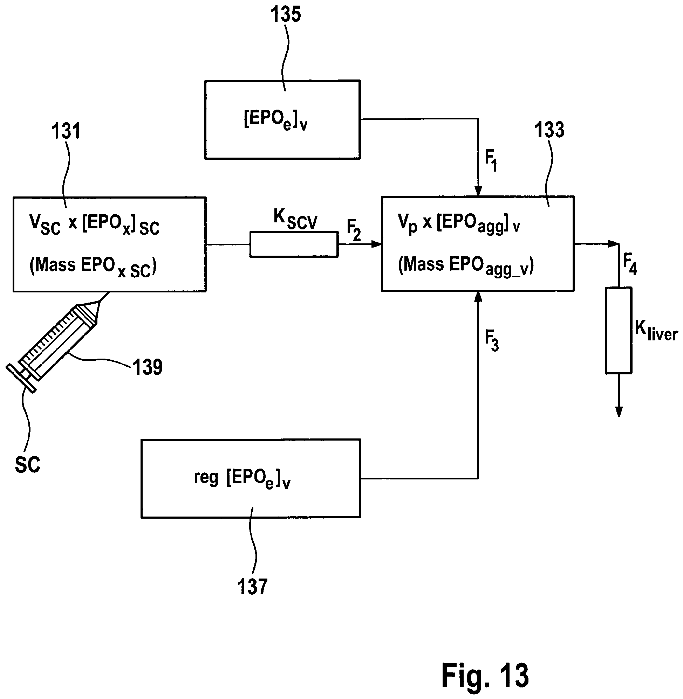

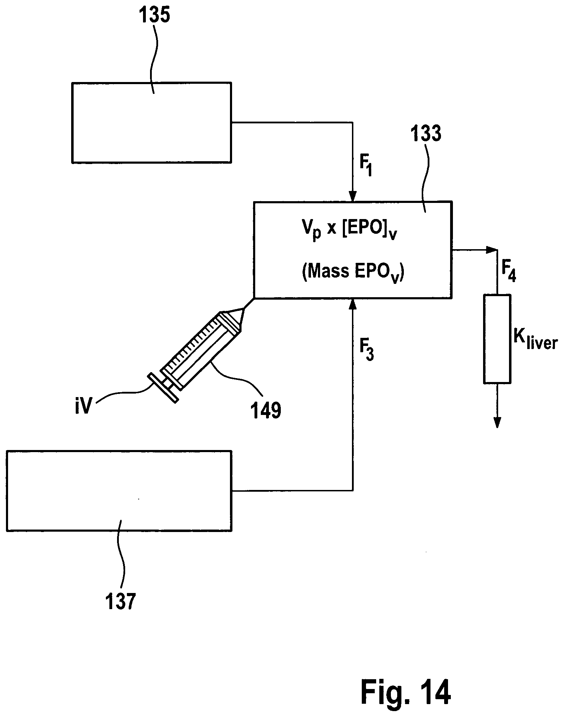

Note that $F_{EPO\_baseline}$ has been shortened to F1 in Figs. 13 and 14.

**Physiologically controlled EPO production**

Wherever it is referred to "physiologically controlled EPO production", the overall production - including the kidney - is contemplated. "Baseline endogenous EPO production" relates, however, to any endogenously produced EPO that stems from any organ (including, for example, the liver) but the kidneys.

Simple proportional control is assumed such that a flux denoted by proportional control factor F3 is generated that is proportional to the difference between the Hb set point and the measured Hb. This may be represented as:

$$F_3 = \left(Hb_{set} - Hb\right) \cdot K_{EPO\_gain} \qquad \text{Eq 19}$$

where $K_{EPO\_gain}$ represents the proportional gain. $K_{EPO\_gain}$ can be easily determined in health as it is the gain that is required to restore Hb back to normal levels within a time frame of x days, following blood loss. Where a subject has partial renal function a reduced value of $K_{EPO\_gain}$ can be expected. This can be easily modeled with the expression:

$$K_{EPO\_gain} = K_{EPO\_Max} \cdot (1 - e^{-k_{rf} \cdot R}) \qquad \text{Eq 20}$$

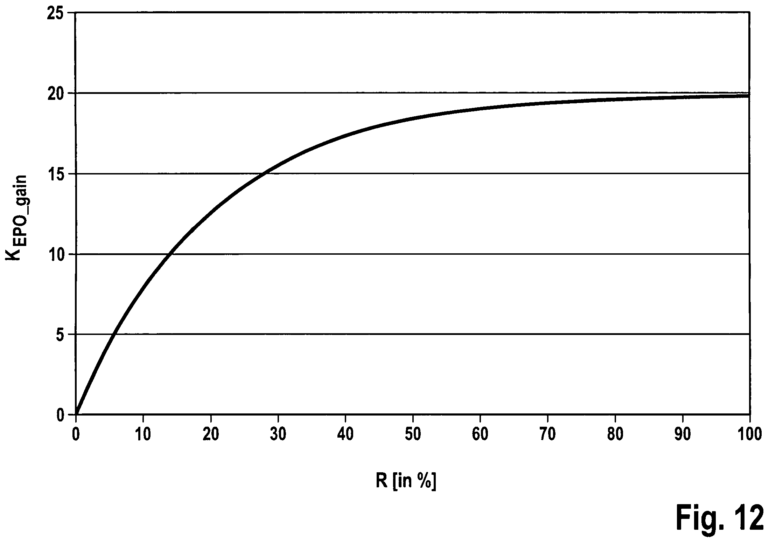

where R (an enhancement, without dimension, see also Fig. 12) is the renal function and $k_{rf}$ is the decay constant.

**Fig. 12** shows the gain of $K_{EPO}$ as a function of the renal function in [%].

**EPO Kinetics 65**

**Subcutaneous administration of EPO**

The derivation of the concentration of EPO appearing in the vascular space applies the scheme shown in **Fig. 13.** The dynamics of the EPO are determined according to two pools namely the subcutaneous compartment 131 and the vascular compartment 133, the mass transfer coefficient Kscv of EPO (from subcutaneous to the vascular space) and the clearance $K_{liver}$ of EPO by the liver. The baseline endogenous EPO concentration $[EPO_e]_v$, see reference numeral 135, is factored in the present model by function F1, its physiological regulation, see reference numeral 137, is factored in the present model by function F2.

For example, the mass transfer coefficient $K_{SCV}$ may be 0.005 ml/min. Although 0.005 ml/min is preferred by the inventors, $K_{SCV}$ is, however, not limited to this value. Rather, any suitable value for $K_{SCV}$ may also be used. Suitable values have been found by the inventors to be in the range between 0.0001 ml/min to 0.1 ml/min, further in the range between 0.001 ml/min and 0.01 ml/min and in the range between 0.0025 ml/min and 0.0075 ml/min.

A steady baseline generation of endogenous EPO is assumed as explained earlier. Note the concentration of EPO is denoted by the use of square brackets to differentiate concentration from mass.

Fig. 13 shows the parameter that influence the subcutaneous administration 139 of EPO according to the 2-pool-model.

The mass of EPO in the vascular compartment 133 is the sum of the endogenous and exogenous components of EPO:

$$M\_EPO_{agg\_v} = \left[EPO_e\right]_v \cdot V_p + \left[EPO_x\right]_v \cdot V_p \qquad \text{Eq 21}$$

To avoid unwieldy expressions in the following derivations, let M = M_EPOagg_v, Ce = [EPOe]v and Cx = [EPOx]v. However, it will be necessary to switch the use of the original variable name and the substitutions for clarity. Rewriting Eq 21 with substitutions made:

$$M = C_e \cdot V_p + C_x \cdot V_p$$

Eq 22

As Ce and Cx and Vp are all variables then a change in the mass of EPO is

$$\delta M = \frac{\partial M}{\partial C_e} \cdot \delta C_e + \frac{\partial M}{\partial C_x} \cdot \delta C_x + \frac{\partial M}{\partial V_p} \cdot \delta V_p$$

Eq 23

From which the rate of change of mass with respect to time is

$$\frac{\delta M}{\delta t} = \frac{\partial M}{\partial C_e} \cdot \frac{\delta C_e}{\delta t} + \frac{\partial M}{\partial C_x} \cdot \frac{\delta C_x}{\delta t} + \frac{\partial M}{\partial V_p} \cdot \frac{\delta V_p}{\delta t}$$

Eq 24

Therefore

$$\frac{\delta M}{\delta t} = V_p \cdot \frac{\delta C_e}{\delta t} + V_p \cdot \frac{\delta C_x}{\delta t} + (C_e + C_x) \cdot \frac{\delta V_p}{\delta t}$$

Eq 25

In the limit as δt→0 then

$$\frac{dM}{dt} = V_p \cdot \frac{dC_e}{dt} + V_p \cdot \frac{dC_x}{dt} + (C_e + C_x) \cdot \frac{dV_p}{dt}$$

Eq 26

Assuming instantaneous mixing of EPO within the vascular compartment, then applying simple mass balance principles, the fluxes of EPO (mass transferred per unit time) is

$$\frac{dM}{dt} = F_1 + F_2 + F_3 - F_4$$

Eq 27

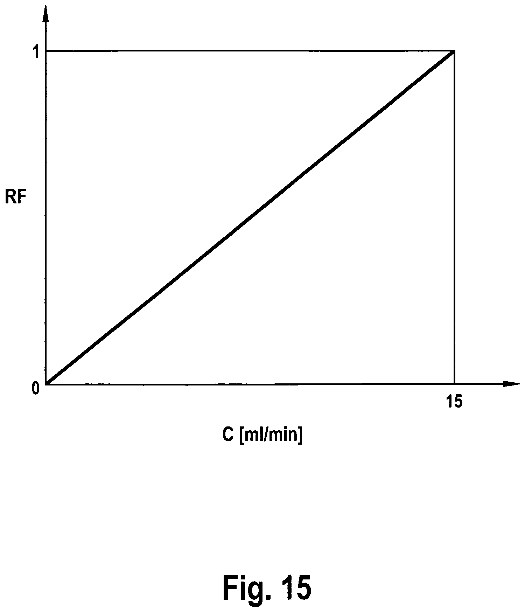

The flux F1 represents the baseline endogenous EPO generation rate. Note that F3 is the case for physiologically regulated EPO flux where some degree of renal function (may be assessed by means of the renal creatinin clearance function, depicted as "C" in **Fig. 15**) is assumed. In chronic renal failure this may be set to zero. The flux F2 depends on the concentration of EPO within the subcutaneous and vascular pools. However, more specifically, it is assumed that the concentration gradient between the two pools is not affected by the EPO composition in the vascular space, but merely the aggregate concentration, i. e.:

$$F_2 = -V_{SC} \cdot \frac{d([EPO_x]_{SC})}{dt} = ([EPO_x]_{SC} - [EPO_{agg}]_V) \cdot K_{SCV}$$

Eq 28

Note the minus signs indicating the direction of flux out of the subcutaneous pool.
The flux through the liver depends on concentration and the clearance rate, but differs depending on the half life of the EPO component. Consequently two liver time constants are defined for endogenous and exogenous components. This may be represented as

$$F_4 = [EPO_e]_v \cdot K_{liver\_e} + [EPO_x]_v \cdot K_{liver\_x}$$

Eq 29

Combining Eq 26 and 27 and rearranging for the endogenous rate of concentration change leads to

$$\frac{dC_e}{dt} = \frac{F_1 + F_2 + F_3 - F_4 - V_p \cdot \frac{dC_x}{dt} - (C_e + C_x) \cdot \frac{dV_p}{dt}}{V_p}$$

Eq 30

and for the exogenous EPO concentration:

$$\frac{dC_x}{dt} = \frac{F_1 + F_2 + F_3 - F_4 - V_p \cdot \frac{dC_e}{dt} - (C_e + C_x) \cdot \frac{dV_p}{dt}}{V_p}$$

Eq 31

and from Eq 28

$$\frac{d[EPO]_{SC}}{dt} = \frac{([EPO]_V - [EPO]_{SC}) \cdot K_{SCV}}{V_{SC}}$$

Eq 32

The half life of endogenous EPO when broken down by the liver is 8 hrs. This may be related to the liver clearance, $K_{liver\_e}$ as follows

$$\frac{K_{liver\_e}}{V_p} = \frac{\ln(2)}{t_{h\_e}}$$

Eq 33

Where the plasma volume Vp is

$$V_p = (1 - Hct) \cdot BV$$

Eq 34

Thus converting to minutes and assuming a blood volume of 5000 ml and Hct of 0.36 then

$$K_{liver\_e} = \frac{\ln(2)}{8 \times 60} \cdot 5000 \cdot (1 - Hct) = 4.62\, ml/\min$$

Eq 35

Exogenous liver clearance $K_{liver\_x}$ can be determined in the same manner. With some ESA agents the half life is 21 hrs leading to a $K_{liver\_x}$ value of 1.76 ml/min. The time of the peak concentration of $[EPOagg]v$ in the blood after EPO administration is governed by the mass transfer coefficient $K_{SCV}$ and the liver clearance. This peak occurs typically 5-24 hours after injection. Taking a value of 12 hours, $K_{SCV}$ can be determined easily from simulation.

**Intravenous administration of EPO**

The scheme for intravenous infusion (iv) is shown in **Fig. 14**. The derivation of the kinetic is similar to that of subcutaneous, but simplified without the presence of a flux F2.

Fig. 14 shows the EPO kinetics of intravenous application (IV). The IV kinetics are identical to subcutaneous kinetics of Fig. 13 with F2 set to zero,

$$\frac{dC_e}{dt} = \frac{F_1 + F_3 - F_4 - V_p \cdot \frac{dC_x}{dt} - (C_e + C_x) \cdot \frac{dV_p}{dt}}{V_p}$$

Eq 36

$$\frac{dC_x}{dt} = \frac{F_1 + F_3 - F_4 - V_p \cdot \frac{dC_e}{dt} - (C_e + C_x) \cdot \frac{dV_p}{dt}}{V_p}$$

Eq 37

F2 is set to zero as it makes no sense to consider a flux of EPO as such, which is administered as an impulse. Instead the effect of IV injection is to reset the [EPOagg]v to a new value caused by the mass of exogenous EPO injected. Thus at the time instant of EPO injection

$$\left[EPO_{agg}\right]_v(t+1) = \frac{\left[EPO_{agg}\right]_v(t) \cdot V_p + \left[EPO_x\right]_{ivi} \cdot V_{inj}}{V_p + V_{inj}}$$

Eq 38

$V_{inj}$ is typically 1 ml for injection. While this crucially important in setting the dose of EPO administered, it clearly has negligible effect on the plasma volume.

## Claims

1. A computer-implemented method for predicting or assessing the concentration or the mass of haemoglobin (Hb) or an approximation thereof, respectively, in a body fluid sample of a patient and/or an extracorporeal sample thereof at a later, second point of time, the patient having theoretically or in reality been administered a certain dose of an erythropoesis stimulating agent (ESA) at an earlier, first point of time, the method comprising the step:

   - predicting or assessing the haemoglobin mass or concentration at the second point of time starting from or based on the corrected mass or concentration value

   **characterized by** the step:

   - measuring and correcting the measured haemoglobin concentration value or an approximation thereof, respectively, in a body fluid sample of a patient and/or an extracorporeal sample thereof, for an overhydration of the patient in order to get a corrected haemoglobin mass or concentration value.

2. A method according to claim 1, wherein a target or target range for assessing haemoglobin mass or concentration values is created based on haemoglobin mass or concentration values corrected for an overhydration of the patient.

3. A method according to claim 1 or 2, wherein at least one factor is mathematically contemplated or considered, selected from a group comprising at least:

   - an endogenous ESA production;
   - a residual renal function;
   - a transferrin saturation;
   - an administration mode of how ESA has been or is to be administered;
   - a rate of erythrocytes produced from pro-erythroblasts present;
   - a factor indicative of the existence and/or degree of physiological endogenous ESA or EPO control;
   - a factor indicative of an iron absorption process;

- a factor indicative of the kinetics of various types of exogenous ESA or EPO;
- a factor reflecting the overhydration of the patient;
- a factor accounting for a dosis schedule;
- a factor accounting for or reflecting ferritin and/or hepsidin; and
- a factor describing functional or absolute iron deficiency.

4. A device for predicting the concentration or the mass of haemoglobin or an approximation thereof, respectively, in a body fluid sample of a patient and/or an extracorporeal sample thereof at a later, second point of time, the patient having theoretically or in reality been administered a certain dose of an erythropoesis stimulating agent at an earlier, first point of time, the device comprising:

- a prediction means configured for predicting the haemoglobin mass or concentration at the second point of time starting from or based on the corrected mass or concentration value;

the device being **characterized by**:

- a correction means configured for correcting a measured haemoglobin concentration value or an approximation thereof, respectively, of the patient, for an overhydration of the patient in order to get a corrected mass or concentration.

5. A device according to claim 4, comprising means for creating a target or target range for assessing haemoglobin mass or concentration values, the target or target range being is created based on haemoglobin mass or concentration values corrected for an overhydration of the patient.

6. A device according to claim 4 or 5, comprising means for mathematically contemplating or considering at least one factor selected from a group comprising at least:

- an endogenous ESA or EPO production;
- a residual renal function;
- a transferrin saturation;
- an administration mode of how ESA or EPO has been or is to be administered;
- a rate of erythrocytes produced from pro-erythroblasts present;
- a factor indicative of the existence and/or degree of physiological endogenous ESA or EPO control;
- a factor indicative of an iron absorption process;
- a factor indicative of the kinetics of various types of exogenous ESA or EPO;
- a factor reflecting the overhydration of the patient;
- a factor accounting for a dosis schedule;
- a factor accounting for or reflecting ferritin or hepsidin; and
- a factor describing functional or absolute iron deficiency.

7. A digital storage means, in particular a disc, CD or DVD, with electrically readable control adapted to interact with a programmable computer system such that a method according to anyone of the preceding method claims will be executed.

**Patentansprüche**

1. Computerimplementiertes Verfahren zur Vorhersage oder zum Bewerten der Konzentration oder der Masse von Hämoglobin (Hb), beziehungsweise einer Annäherung an diese, in einer Körperflüssigkeit eines Patienten und/oder in einer extrakorporalen Probe dieser zu einem späteren, zweiten Zeitpunkt, wobei dem Patienten theoretisch oder tatsächlich eine bestimmte Dosis eines die Erythropoese fördernden Stoffes (ESA) zu einem früheren, ersten Zeitpunkt verabreicht wurde, wobei das Verfahren folgende Schritte umfasst:

- Vorhersagen oder Bewerten der Hämoglobinmasse oder Hämoglobinkonzentration zum zweiten Zeitpunkt, ausgehend von oder basierend auf dem korrigierten Wert der Masse oder der Konzentration

durch den Schritt gekennzeichnet:

- Messen und Korrigieren des gemessenen Hämoglobinkonzentrationswertes, beziehungsweise einer Annäherung an diesen, in einer Körperflüssigkeit eines Patienten und/oder in einer extrakorporalen Probe dieser für eine Überwässerung des Patienten, um einen korrigierten Hämoglobinmassen- oder Hämoglobinkonzentrationswert zu erhalten.

2. Das Verfahren nach Anspruch 1, wobei ein Zielwert oder ein Zielwertbereich zum Bewerten von Hämoglobinmassen- oder Hämoglobinkonzentrationswerten erzeugt wird, basierend auf Hämoglobinmassen- oder Hämoglobinkonzentrationswerten, die für eine Überwässerung des Patienten korrigiert sind.

3. Das Verfahren nach Anspruch 1 oder 2, wobei mindestens ein Faktor mathematisch in Erwägung gezogen oder berücksichtigt wird, der aus einer Gruppe ausgewählt ist, die mindestens umfasst:

   - eine endogene ESA-Produktion;
   - eine residuelle Nierenfunktion,
   - eine Transferrin-Sättigung;
   - eine Verabreichungsart, nach der ESA verabreicht wurde oder verabreicht werden soll;
   - eine Rate von Erythrozyten, die von vorhandenen Pro-Erythroblasten produziert wurden;
   - ein Faktor, der für die Existenz und/oder das Maß an physiologischem endogenem ESA oder EPO-Kontrolle bezeichnend ist;
   - ein Faktor, der bezeichnend für einen Eisenabsorptionsprozess ist;
   - ein Faktor, der für die Kinetik unterschiedlicher Typen von exogenem ESA oder EPO bezeichnend ist;
   - ein Faktor, der die Überwässerung des Patienten reflektiert;
   - ein Faktor, der ein Dosierschema berücksichtigt;
   - ein Faktor, der Ferritin und/oder Hepcidin berücksichtigt oder reflektiert; und
   - ein Faktor, der funktionellen oder absoluten Eisenmangel beschreibt.

4. Vorrichtung zum Vorhersagen der Konzentration oder der Masse von Hämoglobin, beziehungsweise einer Annäherung daran, in einer Körperflüssigkeit eines Patienten und/oder in einer extrakorporalen Probe dieser zu einem späteren, zweiten Zeitpunkt, wobei dem Patienten theoretisch oder tatsächlich eine bestimmte Dosis eines die Erythropoese fördernden Stoffes zu einem ersten, früheren Zeitpunkt verabreicht wurde, wobei die Vorrichtung umfasst:

   - ein Vorhersagemittel, das konfiguriert ist, um die Hämoglobinmasse oder die Hämoglobinkonzentration zum zweiten Zeitpunkt vorherzusagen, ausgehend von oder basierend auf dem korrigierten Massen- oder Konzentrationswert;

   wobei die Vorrichtung durch

   - ein Korrekturmittel, das konfiguriert ist, um einen gemessenen Hämoglobinkonzentrationswert des Patienten beziehungsweise eine Annäherung an diesen für die Überwässerung des Patienten zu korrigieren, um eine korrigierte Masse oder Konzentration zu erhalten

   gekennzeichnet ist.

5. Die Vorrichtung nach Anspruch 4 umfassend Mittel zum Erzeugen eines Zielwerts oder Zielwertbereichs zum Bewerten von Hämoglobinmassen- oder -konzentrationswerten, wobei der Zielwert oder der Zielwertbereich, der erzeugt wird, auf Hämoglobinmassen- oder Hämoglobinkonzentrationswerten basiert, die für eine Überwässerung des Patienten korrigiert sind.

6. Die Vorrichtung nach Anspruch 4 oder 5 umfassend Mittel zum mathematischen Erwägen oder Berücksichtigen mindestens eines Faktors, der aus einer Gruppe ausgewählt ist, die mindestens umfasst:

   - eine endogene ESA- oder EPO-Produktion;
   - eine residuelle Nierenfunktion,
   - eine Transferrin-Sättigung
   - eine Verabreichungsart, nach der ESA oder EPO verabreicht wurde oder verabreicht werden soll;
   - eine Rate von Erythrozyten, die von vorhandenen Pro-Erythroblasten produziert wurden;
   - einen Faktor, der für die Existenz und/oder das Maß an physiologischem endogenem ESA oder EPO-Kontrolle bezeichnend ist;
- einen Faktor, der bezeichnend für einen Eisenabsorptionsprozess ist;
- einen Faktor, der für die Kinetik unterschiedlicher Typen von exogenem ESA oder EPO bezeichnend ist;
- einen Faktor, der die Überwässerung des Patienten reflektiert;
- einen Faktor, der ein Dosierschema berücksichtigt;
- einen Faktor, der Ferritin und/oder Hepcidin berücksichtigt oder reflektiert; und
- einen Faktor, der funktionellen oder absoluten Eisenmangel beschreibt.

7. Ein digitales Speichermedium, insbesondere in Form einer Diskette, CD oder DVD, mit elektronisch auslesbarem Steuer, geeignet, um derart mit einem programmierbaren Computersystem zusammenzuwirken, dass ein Verfahren nach einem der vorangehenden Verfahrensansprüche veranlasst wird.

**Revendications**

1. Procédé mis en oeuvre par ordinateur pour prédire ou évaluer la concentration ou la masse d'hémoglobine (Hb), ou une approximation de celle-ci, respectivement, dans un fluide corporel d'un patient et / ou dans un échantillon extracorporel de celui-ci, à un second moment ultérieur, le patient ayant théoriquement ou en réalité reçu une certaine dose d'un agent stimulant l'érythropoïèse (ESA) à un premier moment antérieur, le procédé comprenant l'étape consistant à :

   - prédire ou évaluer la masse ou la concentration d'hémoglobine, au second moment à partir de ou en fonction de la valeur corrigée de la masse ou de la concentration

   le procédé étant **caractérisé par** l'étape consistant à:

   - mesurer et corriger une valeur mesurée de la concentration de l'hémoglobine, ou une approximation de celle-ci, respectivement, dans un fluide corporel d'un patient et / ou dans un échantillon extracorporel de celui-ci pour une hyperhydratation du patient afin d'obtenir une valeur corrigée de la masse ou de la concentration d'hémoglobine.

2. Le procédé selon la première revendication, dans lequel une valeur cible ou un éventail de valeurs cibles est créé(e) sur la base des valeurs de masse ou de concentration de l'hémoglobine corrigées pour une hyperhydratation du patient.

3. Le procédé selon la première ou la seconde revendication, dans lequel au moins un facteur est mathématiquement envisagé ou considéré, lequel est choisi d'un groupe comprenant au moins :

   - une production de l'ESA endogène ;
   - une fonction rénale résiduelle ;
   - une saturation de la transferrine ;
   - un mode d'administration de la façon dont l'ESA a été ou doit être administré ;
   - un taux d'érythrocytes produits à partir des pré-érythroblastes présents ;
   - un facteur indicatif de l'existence et / ou du degré de l'ESA endogène physiologique ou du contrôle de l'EPO ;
   - un facteur indicatif d'un processus d'absorption du fer ;
   - un facteur indicatif de la cinétique de divers types d'ESA ou d'EPO exogènes ;
   - un facteur reflétant l'hyperhydratation du patient ;
   - un facteur tenant compte d'un schéma posologique ;
   - un facteur tenant compte ou reflétant la ferritine et / ou l'hepcidine ; et
   - un facteur décrivant une insuffisance fonctionnelle ou absolue en fer.

4. Un dispositif pour la prédiction de la concentration ou de la masse d'hémoglobine, ou une approximation de celle-ci, respectivement, dans un fluide corporel d'un patient et / ou dans un échantillon extracorporel de celui-ci, à un second moment ultérieur, le patient ayant théoriquement ou en réalité reçu une certaine dose d'un agent stimulant l'érythropoïèse à un premier moment antérieur, le dispositif comprenant :

   - un moyen de prédiction conçu pour prédire la masse ou la concentration d'hémoglobine dans un second temps, à partir de ou sur la base de la valeur corrigée de la masse ou de la concentration ;

le dispositif étant **caractérisé par**:

- un moyen de correction conçu pour corriger une valeur mesurée de la concentration d'hémoglobine du patient, ou une approximation de celle-ci, respectivement, pour une hyperhydratation du patient afin d'obtenir une masse ou une concentration corrigée.

5. Le dispositif selon la revendication 4, comprenant des moyens pour créer une valeur cible ou un éventail de valeurs cibles afin d'évaluer des valeurs de masse ou de concentration d'hémoglobine, la valeur cible ou l'éventail de valeurs cibles étant créé(e) sur la base des valeurs de masse ou de concentration d'hémoglobine corrigées pour une hyperhydratation du patient.

6. Le dispositif selon la revendication 4 ou 5, comprenant des moyens pour envisager ou considérer mathématiquement au moins un facteur choisi dans un groupe comprenant au moins :

- une production de l'ESA ou de l'EPO endogène ;
- une fonction rénale résiduelle ;
- une saturation de la transferrine ;
- un mode d'administration de la façon dont l'ESA ou l'EPO a été ou doit être administré ;
- un taux d'érythrocytes produits à partir des pro-érythroblastes présents ;
- un facteur indicatif de l'existence et / ou du degré de l'ESA endogène physiologique ou du contrôle de l'EPO ;
- un facteur indicatif d'un processus d'absorption du fer ;
- un facteur indicatif de la cinétique de divers types d'ESA ou d'EPO exogènes ;
- un facteur reflétant l'hyperhydratation du patient ;
- un facteur tenant compte d'un schéma posologique ;
- un facteur tenant compte ou reflétant la ferritine et / ou l'hepcidine ; et
- un facteur décrivant une insuffisance fonctionnelle ou absolue en fer.

7. Un dispositif de stockage numérique, en particulier un disque, CD ou DVD, avec une commande lisible électriquement adaptée pour interagir avec un système informatique programmable, de manière à ce qu'un procédé, selon l'une quelconque des revendications précédentes relatives à un procédé, soit exécuté.

**Fig. 1**

**Fig. 2**

**Fig. 3**

Fig. 4

Fig. 5

$$\left[ \dfrac{g(Hb)}{d} \right]$$

Fig. 6

[d]

$$G_{Hb}(t=4) = \sum_{i=1}^{N} g_i \ (t=4)$$

$$G_{Hb}(t=3) = \sum_{i=1}^{N} g_i \ (t=3)$$

$$G_{Hb}(t=2) = \sum_{i=1}^{N} g_i \ (t=2)$$

$$G_{Hb}(t=1) = \sum_{i=1}^{N} g_i \ (t=1)$$

I = 1, 2, ... N

## Fig. 7

**Fig. 8**

EP 2 550 531 B1

Fig. 9

Fig. 10

EP 2 550 531 B1

Fig. 11

Fig. 12

**Fig. 13**

**Fig. 14**

**Fig. 15**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006002685 A1

- WO 9219153 A

### Non-patent literature cited in the description

- Replacement of Renal Function by Dialysis. **DRUKKER ; PARSON ; MAHER.** Hemodialysis machines and monitors. Kluwer Academic Publisher, 2004, 397-401